# EUROPÄISCHE PATENTSCHRIFT

(11) **EP 2 225 208 B1**
(45) Veröffentlichungstag und Bekanntmachung des Hinweises auf die Patenterteilung: **18.06.2014**
(21) Anmeldenummer: 08863783.0
(22) Anmeldetag: 10.12.2008
(51) Int. Cl.: C07D 233/34

(54) **Dauerhaft rieselfähiger Ethylenharnstoff**
Permanently free-flowing ethylene urea
Éthylène urée durablement versable

(30) Priorität: 21.12.2007 EP 07150393
(43) Veröffentlichungstag der Anmeldung: 08.09.2010
(73) Patentinhaber: BASF SE, 67056 Ludwigshafen (DE)
(72) Erfinder: SZAFRANSKI, Henry, 68219 Mannheim (DE); WERLAND, Stefanie, 68305 Mannheim (DE); RAATZ, Peter, 67069 Ludwigshafen (DE); SIMON, Joachim, 67063 Ludwigshafen (DE)
(86) Internationale Anmeldenummer: PCT/EP2008/067193
(87) Internationale Veröffentlichungsnummer: WO 2009/080513

(56) Entgegenhaltungen:
- DE-A1-102005 054 462
- US-A- 2 436 311
- US-A- 2 497 309
- US-A- 2 504 431
- US-A- 2 526 757
- US-A- 2 993 906

## Beschreibung

Die vorliegende Erfindung betrifft ein Verfahren zur Herstellung von Ethylenharnstoff in fester Form, wobei eine wasserhaltige Produktschmelze von Ethylenharnstoff auf einer Vereinzelungseinrichtung gekühlt wird und der Wassergehalt des erhaltenen Feststoffs von 5 bis 15 Gew.-% beträgt.

Ethylenharnstoff (2-Imidazolidon, Imidazolidin-2-on) wird in der Prozesstechnik vielfältig eingesetzt. Als Zwischenprodukt ist er unter anderem für die Pharmaindustrie von Bedeutung. Ethylenharnstoff findet weiterhin Verwendung bei der Herstellung von Weichmachern, Lacken, Polymeren sowie Textil- und Lederhilfsmitteln. Die Verarbeitung erfolgt je nach Einsatzgebiet kontinuierlich oder absatzweise, beispielsweise in Rührreaktoren.

Die Herstellung von Ethylenharnstoff aus unterschiedlichen Ausgangsmaterialien ist seit geraumer Zeit bekannt. In den Patentschriften US 2,436,311, US 2,504,431 und US 2,526,757 wird die Herstellung von Ethylenharnstoff aus Ethylendiamin und Harnstoff in Gegenwart von Wasser vorgeschlagen. Der erhaltene Ethylenharnstoff wird vom Rohprodukt abgetrennt, von Wasser und Resten von Ethylendiamin befreit und anschließend über eine Schuppenwalze gefahren. Die Herstellung und Verpackung der Schuppen muss dabei unter Luftausschluss geschehen. Alternativ kann das Produkt in einem geeigneten Lösungsmittel kristallisiert werden.

Die Patentschrift US 2,497,309 beschreibt ein Verfahren, das den oben genannten entspricht, mit dem Unterschied, dass anstelle von Harnstoff Kohlendioxid als Einsatzstoff verwendet wird.

In US 2,751,394 wird zur Verbesserung der Farbstabilität des fertigen Produkts der Zusatz von Stabilisatoren, beispielsweise Zitronensäure, zum Ethylenharnstoff vorgeschlagen. Der Ethylenharnstoff kann dabei aus Ethylendiamin und Harnstoff hergestellt worden sein. Das Endprodukt kann entweder als wässrige Lösung vorliegen oder in fester Form, z.B. als Schuppen. Auch gemäß diesem Verfahren wird der Ethylenharnstoffschmelze im Wesentlichen alles Wasser entzogen.

In US 2,993,906 wird die Reinigung von Imidazolidonen, insbesondere von Ethylenharnstoff, offenbart mit dem Ziel, einen weißen, geruchlosen und kristallinen Feststoff zu erhalten. Dies wird erreicht, indem eine wässrige Lösung von Ethylenharnstoff mit einem Ionentauscherharz in Kontakt gebracht wird. Aus der wässrigen Lösung wird eine klare Schmelze erhalten, die durch Abkühlung zu einer kristallinen Masse wird. Diese Masse wird zu einem feinen Pulver vermahlen und getrocknet. Das Produkt enthält nicht näher bestimmte Mengen des zur Reinigung eingesetzten lonentauscherharzes.

Die Anmeldung DE 10 2005 054 462 A1 beschreibt ein Verfahren zum Pastillieren von Harnstoff und eine dafür geeignete Pastillieranlage. Durch Auftragen eines dünnen Produkt-Lösemittel-Filmes lässt sich auch für hygroskopische Produkte selbst bei hoher Luftfeuchtigkeit eine gleichbleibende Pastillenqualität sicherstellen. Die Produkte sind dabei im Wesentlichen trocken und nehmen während der Verarbeitung nur eine sehr geringe Menge an Flüssigkeit auf.

Aufgabe des erfindungsgemäßen Verfahrens war es, einen kostengünstigen Weg zur Herstellung eines dauerhaft rieselfähigen, nicht staubenden Ethylenharnstoffs aufzuzeigen. Insbesondere sollte das Produkt auch nach mehrmonatiger Lagerung im geschlossenen Gebinde, beispielsweise in einem Sack oder einem Fass, durch einfaches Kippen aus diesem entleert werden können, ohne dass mechanische Hilfsmittel zur Auflockerung eingesetzt werden müssen. Eine weitere Aufgabe der vorliegenden Erfindung war es, ein weißes bis blassgelbes Produkt zur Verfügung zu stellen. Insbesondere sollte sich das Produkt auch während der Lagerung nicht oder nicht wesentlich verfärben.

Diese Aufgabe wird durch die Gegenstände der Erfindung gelöst. Ein Gegenstand der Erfindung ist ein Verfahren zur Herstellung von Ethylenharnstoff in fester Form, wobei eine Produktschmelze von Ethylenharnstoff mit einem Wassergehalt von 5 bis 15 Gew.-% mittels einer Vereinzelungseinrichtung gekühlt wird und der erhaltene Feststoff im Wesentlichen denselben Wassergehalt aufweist wie die Produktschmelze.

Gemäß der Erfindung ist Ethylenharnstoff in fester Form erhältlich nach einem der erfindungsgemäßen Verfahren, wobei der Wassergehalt im Feststoff 5 bis 15 Gew.-% beträgt.

Nach einem erfindungsgemäßen Verfahren hergestellter Ethylenharnstoff ist vielfältig einsetzbar, beispielsweise in chemischen Synthesen als Reaktionspartner oder als Mischungspartner. Ethylenharnstoff dient unter anderem zur Herstellung von Weichmachern, Lacken und Pharmazeutika. Der gemäß eines erfindungsgemäßen Verfahrens hergestellten Ethylenharnstoffs kann zur Herstellung von Lacken sowie Textil- und Lederhilfsmitteln verwendet werden. Bei der Lackherstellung sowie in der Textil- und Lederindustrie wird Ethylenharnstoff insbesondere als Formaldehydfänger eingesetzt.

Bevorzugte Ausführungsformen sind den Ansprüchen und der Beschreibung zu entnehmen. Kombinationen bevorzugter Ausführungsformen sind von der Erfindung mit umfasst.

Großtechnisch kann Ethylenharnstoff durch Umsetzung von 1,2-Ethylendiamin mit Harnstoff hergestellt werden, indem diese beiden Ausgangsstoffe vermischt und auf eine Temperatur von mindestens 260°C erhitzt werden. Dabei entsteht eine Rohschmelze, die neben der gewünschten Verbindung und Ammoniak auch wasserunlösliche Nebenprodukte enthält. Üblicherweise wird die Rohschmelze rasch mit einem geeigneten Lösungsmittel, vorzugsweise Wasser, auf Temperaturen um 150°C abgeschreckt. Dadurch erhält man eine wässrige Lösung mit einem Ethylenharnstoffanteil von beispielsweise etwa 80%. Die wässrige Lösung wird in der Regel von den wasserunlöslichen Nebenkomponenten befreit, beispielsweise durch Filtration. Das Wertprodukt Ethylenharnstoff wird im Allgemeinen in einem weiteren Verfahrensschritt durch Kristallisation aus der wässrigen Lösung gewonnen. Die Kristalle können mit bekannten Verfahren von der Lösung getrennt werden, beispielsweise durch Abschleudern in einer Siebschneckenzentrifuge. Der so erzeugte kristallisierte Ethylenharnstoff enthält noch etwa 5 bis 15 Gew.-%, z.B. 8 bis 12 Gew.-%, meist ca. 10 Gew.-% Wasser.

Wird dieses Produkt gelagert, so verbinden sich die feuchten Kristalle miteinander, und die Masse verklumpt innerhalb weniger Stunden zu einem festen Block. Diese Eigenschaft des handelsüblichen Ethylenharnstoffs bewirkt, dass das Produkt beim Anwender beispielsweise nicht ohne Weiteres in einen Behälter dosiert werden kann, sondern vor weiterer Verwendung mechanisch zerkleinert werden muss.

Eine Möglichkeit, das Verklumpen zu verhindern, besteht in der Trocknung des feuchten Kristallisats in einem geeigneten Trockenapparat, beispielsweise einem Schaufeltrockner. Dieser Schritt ist vergleichsweise aufwändig und liefert ein feinpulveriges, staubendes Produkt, das nur schwer in einen Batch-Ansatz eingerührt werden kann, da es auf der Oberfläche aufschwimmt.

Überraschend wurde gefunden, dass das Verklumpen durch ein alternatives Verfahren vermieden werden kann, das im Folgenden näher ausgeführt wird.

Erfindungsgemäß wird eine wasserhaltige Produktschmelze von Ethylenharnstoff mittels einer Vereinzelungseinrichtung gekühlt und in einen Feststoff überführt. Die wasserhaltige Produktschmelze lässt sich auf unterschiedliche Arten erzeugen. In einer vorteilhaften Ausführungsform wird der kristallisierte Ethylenharnstoff bei Temperaturen von 50 bis 120°C, bevorzugt 80 bis 100°C aufgeschmolzen. In Abhängigkeit von der in den Kristallen eingeschlossenen Wassermenge erhält man so eine Produktschmelze, die 85 bis 95 Gew.-%, z.B. 88 bis 92 Gew.-%, meist ca. 90 Gew.-% Ethylenharnstoff enthält. Um den Wassergehalt der Produktschmelze im erfindungsgemäßen Bereich sicherzustellen, können weitere Verfahrensschritte vorgesehen werden, beispielsweise eine Destillation. Im Allgemeinen sind solche zusätzlichen Verfahrensschritte allerdings nicht erforderlich.

Bei einer weiteren vorteilhaften Variante wird die aus der Reaktion erhaltene Rohschmelze mit Wasser abgeschreckt, beispielsweise indem man die Rohschmelze in eine Wasservorlage einbringt oder Wasser in die Schmelze einbringt. Die Schmelze wird dadurch im Allgemeinen auf eine Temperatur von 50 bis 120°C, bevorzugt 80 bis 100°C gebracht. Wasserunlösliche Nebenprodukte können von der Schmelze abgetrennt werden, beispielsweise mit herkömmlichen Verfahren wie Filtration.

Die nach einer der beiden beschriebenen Varianten hergestellte Produktschmelze weist einen Wassergehalt von 2 bis 18 Gew.-%, bevorzugt von 5 bis 15 Gew.-%, besonders bevorzugt von 8 bis 12 Gew.-% auf.

Für das erfindungsgemäße Verfahren eignet sich prinzipiell jede Vereinzelungseinrichtung, die gekühlt werden kann. In einer bevorzugten Ausführungsform besteht die Vereinzelungseinrichtung im Wesentlichen aus einer Schuppenwalze. Bei einer Schuppenwalze handelt es sich üblicherweise um einen hohlen Metallzylinder, der von innen mittels eines Kühlmediums, vorzugsweise Wasser, gekühlt wird. Die Temperatur auf der Außenseite der Walze beträgt dabei bevorzugt von 10 bis 30°C. Die Produktschmelze kann auf verschiedene Arten mit der Walze in Kontakt gebracht werden. Häufig wird die Schmelze in einer beheizbaren Wanne vorgelegt. Die Walze taucht teilweise in die Wanne ein und nimmt während der Drehung kontinuierlich Produkt auf. Das Produkt kann auch durch eine separate, typischerweise kleinere Aufnehmerwalze auf die Schuppenwalze aufgebracht werden. Eine Alternative zum Eintauchen in eine Wanne stellt die Zuführung seitlich oder von oben auf die Schuppenwalze dar. Auch ein System von zwei gegenläufigen Schuppenwalzen ist möglich, bei dem die Schmelze beispielsweise an der Stelle zugeführt wird, an der die beiden Walzen ihren geringsten Abstand aufweisen. Die Produktschmelze wird auf der Walze gekühlt und erstarrt. Mittels eines Abnehmers, beispielsweise eines Abstreifers oder Messers, wird das erstarrte Material von der Schuppenwalze abgenommen. Dabei entstehen üblicherweise Schuppen unregelmäßiger Form. Durch geeignete Wahl des Abnehmers können auch Filme, Körnchen, Nadeln oder andere geometrische Formen erhalten werden. Betriebsparameter wie Filmdicke oder Rotationsgeschwindigkeit der Walze können von einem Fachmann nach bekannten Methoden ausgelegt werden.

Eine Alternative der erfindungsgemäßen Vereinzelungseinrichtung stellen Schuppenbänder dar. Sie bestehen üblicherweise aus einem umlaufenden dünnen Band, beispielsweise aus einem Edelstahlband von etwa 1 mm Stärke. Die obere Lage des Bandes wird in der Regel von unten mit einem Kühlmedium, meist Wasser, besprüht, sodass die Bandoberfläche bevorzugt eine Temperatur von 10 bis 30°C aufweist. Die Produktschmelze kann auf unterschiedliche Arten auf das Band aufgebracht werden. Eine Möglichkeit der Zuführung besteht in einem System aus zwei gegenläufigen Walzen, in deren Mitte die Schmelze aufgegeben wird. Über den Abstand der Walzen, ihre Rotationsgeschwindigkeit und die Geschwindigkeit des Bandes kann die Dicke der auf das Band aufgebrachten Schmelze eingestellt werden. Alternativ kann die Schmelze mittels einer Wanne mit Überlauf gleichmäßig auf das Band aufgegeben werden. Die Schmelze erstarrt auf dem Band und wird typischerweise an der Umlenkung des Bandes mittels eines Abnehmers vom Band entfernt. Der Abnehmer kann ein Abstreifer, Schaber oder auch eine andere Einrichtung sein, die geeignet ist, das erstarrte Material von dem Band zu lösen. Der Abnehmer kann auch derart ausgestaltet sein, dass er das erstarrte Material zerkleinert. Ein Beispiel hierfür ist ein Walzensystem, das vom Band abgelöste Platten in Schuppen bricht.

Eine weitere Ausführungsform der erfindungsgemäßen Vereinzelungseinrichtung ist ein Pastillierband von ähnlicher Bauform, wie sie in DIE 10 2005 054 462 A1 ausgeführt ist. Die Funktionsweise ist mit der eines oben beschriebenen Schuppenbandes vergleichbar. Allerdings wird die Produktschmelze nicht in Form eines Filmes auf das Band aufgegeben, sondern als einzelne Tropfen, die aufgrund der Kühlung des Bandes zu Pastillen erstarren. Die Formung der Tropfen geschieht häufig mittels eines Rotor-Stator-Systems, bei dem sich ein perforierter äußerer Zylinder um einen inneren Stator dreht. Die Produktschmelze wird dabei über den Stator von innen auf den perforierten Zylinder aufgegeben. Durch die Löcher läuft die Schmelze auf das Band und wird aufgrund der Rotation des äußeren Zylinders in einzelne Tropfen abgeschert.

Eine ausführliche Übersicht zu Pastillierbändern, Schuppenbändern und Schuppenwalzen sowie deren Auslegung findet sich in dem Buch von C.M. Van't Land: Industrial crystallization of melts, Marcel Dekker, New York, 2005 (Kapitel 3 und 4, S. 45-116).

Die entstehenden Schuppen oder Pastillen weisen im Wesentlichen denselben Wassergehalt auf wie die Produktschmelze. Form und Größe der Schuppen hängen von der verwendeten Vereinzelungseinrichtung ab. Typischerweise sind die Schuppen unregelmäßig geformt. Ihre Länge und Breite betragen im Allgemeinen jeweils von 0,5 bis 3 cm, bevorzugt von 1 bis 2 cm. Die Dicke der Schuppen beträgt in der Riegel von 0,1 bis 3 mm, bevorzugt von 0,2 bis 2 mm. Die Pastillen weisen bevorzugt einen im Wesentlichen kreisrunden Querschnitt von 5 bis 10 mm auf, sind unten eben und nach oben konvex gewölbt.

Der pH-Wert der in Wasser gelösten Ethytenharnstoff-Schuppen oder -Pastillen liegt typischerweise zwischen 10 und 11, bevorzugt bei 10,5, gemessen in einer 10-prozentigen Ethylenharnstoff-Lösung bei 20°C. Die Schuppen fühlen sich trocken an. Wird die Produktschmelze aus kristallisierter Ware gewonnen, sind die Schuppen oder Pastillen weiß mit einer Farbzahl kleiner 80 APHA, bevorzugt kleiner 50 APHA, besonders bevorzugt kleiner 20 APHA. Die Schuppen oder Pastillen ergeben eine klare Lösung, wenn sie in Wasser eingerührt werden. Weder die Schuppen oder Pastillen selbst noch die wässrige Lösung haben einen nennenswerten Geruch.

Erzeugt man die Produktschmelze direkt aus der Rohschmelze, wie in einer alternativen Variante beschrieben, sind die Schuppen oder Pastillen weiß bis hell gelblich mit einer Farbzahl kleiner 250 APHA, bevorzugt kleiner 200 APHA, besonders bevorzugt kleiner 150 APHA. In Wasser eingerührt stellt sich eine leichte Trübung ein. Die Schuppen oder Pastillen und ihre wässrige Lösung können leicht ammoniakalisch riechen.

Im Gegensatz zu der kristallisierten Ware verbackt der nach dem erfindungsgemäßen Verfahren hergestellte Ethylenharnstoff in fester Form, insbesondere die Schuppen oder Pastillen, nicht und ist auch nach mehreren Monaten Lagerung in festen Gebinden wie Säcken oder Fässern noch rieselfähig. Dies ist umso überraschender, als die Schuppen oder Pastillen im Wesentlichen denselben Wassergehalt aufweisen wie die kristallisierte Ware.

Die folgenden Beispiele dienen der näheren Erläuterung des erfindungsgemäßen Verfahrens.

### Beispiel 1:

2500 kg kristallisierter Ethylenharnstoff wurden in einen heizbaren Rührbehälter eingebracht, auf 80°C erhitzt und so in ihrem eigenen Kristallwasser geschmolzen. Diese Produktschmelze wurde in Mengen von 100 bis 300 kg/h direkt auf eine mit Wasser auf 25 bis 33°C gekühlte Schuppenwalze gefahren. Man erhielt 2440 kg weiße, ca. 0,5 bis 1 mm dicke, unregelmäßig geformte Schuppen von Ethylenharnstoff. Länge und Breite betrugen jeweils etwa 1 bis 2 cm. Die Schuppen fühlten sich trocken an, hatten aber denselben Wassergehalt wie die kristallisierte Ware, da zwischen Schmelzen und Verschuppen kein Verfahrensschritt geschaltet war, in dem das Wasser hätte abgetrennt werden können.

### Beispiel 2:

4000 kg einer heißen, aus der Umsetzung von Ethylendiamin mit Harnstoff erhaltenen Schmelze von rohem Ethylenharnstoff wurden in einem Rührbehälter so schnell wie möglich mit 400 kg Wasser versetzt und auf 80°C heruntergekühlt. Die so erhaltene Produktschmelze wurde bei 80°C erst mehrfach über einen Zentrifugalscheibenfilter filtriert und anschließend mit einem Mengenstrom von 100 bis 300 kg/h auf eine mit Wasser auf 25 bis 33°C gekühlte Schuppenwalze gefahren. Man erhielt 4200 kg weiße bis blassgelbe, ca. 0,5 bis 1 mm dicke, unregelmäßig geformte Schuppen von Ethylenharnstoff. Länge und Breite betrugen jeweils etwa 1 bis 2 cm. Trotz eines Wassergehaltes von etwa 9 Gew.-% fühlten sich die Schuppen trocken an.

In beiden Beispielen wurde eine Schuppenwalze aus Stahl eingesetzt mit einer Breite von 2000 mm und einem Durchmesser von 1000 mm. Die Rotationsgeschwindigkeit bei der Verschuppung betrug 1 bis 2 U/min, wobei die Ethylenharnstoffschmelze erst langsam, dann zunehmend schneller auf die Schuppenwalze aufgebracht wurde. Der Ethylenharnstoff-Film auf der Schuppenwalze war etwa 1 mm dick.

Der Mengenunterschied zwischen Einsatzstoffen und erhaltenem Produkt von 60 bzw. 200 kg in den obigen Beispielen rührt daher, dass jeweils eine leere, saubere Schuppenwalze eingesetzt wurde, und nach Beendigung der Versuche entsprechende Restmengen in der Einrichtung verblieben sind. Bei einem kontinuierlichen Betrieb der Schuppenwalze würden sich diese Restmengen nicht signifikant ändern.

Die Fässer (70 kg - Gebinde aus PE) wurden eingelagert und jeweils nach einwöchiger, dreimonatiger, sechsmonatiger und einjähriger Lagerung geöffnet. Die Fässer ließen sich auch nach einjähriger Lagerung durch Umkippen problemlos entleeren. Auch die unten im Fass liegenden Schuppen waren durch den Druck des aufliegenden Materials nicht nennenswert verfestigt worden. Eine Änderung der Farbzahl und des Wassergehalts der Schuppen wurde bei der Lagerung in den dicht verschlossenen Gebinden nicht beobachtet.

## Patentansprüche

1. Verfahren zur Herstellung von Ethylenharnstoff in fester Form, wobei eine Produktschmelze von Ethylenharnstoff mit einem Wassergehalt von 5 bis 15 Gew.-% mittels einer Vereinzelungseinrichtung gekühlt wird und der erhaltene Feststoff im Wesentlichen den gleichen Wassergehalt aufweist wie die Produktschmelze.

2. Verfahren nach Anspruch 1, **dadurch gekennzeichnet, dass** die Produktschmelze und der Feststoff einen Wassergehalt von 8 bis 12 Gew.-% aufweisen.

3. Verfahren nach Anspruch 1 oder 2, **dadurch gekennzeichnet, dass** die wasserhaltige Produktschmelze gewonnen wird, indem kristallisierter Ethylenharnstoff aufgeschmolzen wird.

4. Verfahren nach Anspruch 1 oder 2, **dadurch gekennzeichnet, dass** die wasserhaltige Produktschmelze gewonnen wird, indem eine Rohschmelze des Ethylenharnstoffs mit Wasser versetzt wird.

5. Verfahren nach mindestens einem der Ansprüche 1 bis 4, **dadurch gekennzeichnet, dass** es sich bei der Vereinzelungseinrichtung um eine Schuppenwalze oder ein Schuppenband handelt.

6. Verfahren nach mindestens einem der Ansprüche 1 bis 4, **dadurch gekennzeichnet, dass** es sich bei der Vereinzelungseinrichtung um ein Pastillierband handelt.

## Claims

1. A process for preparing ethylene urea in solid form, wherein a product melt of ethylene urea having a water content of from 5 to 15% by weight is cooled by means of a breaking-up apparatus and the solid obtained has essentially the same water content as the product melt.

2. The process according to claim 1, wherein the product melt and the solid have a water content of from 8 to 12% by weight.

3. The process according to claim 1 or 2, wherein the water-comprising product melt is obtained by melting crystallized ethylene urea.

4. The process according to claim 1 or 2, wherein the water-comprising product melt is obtained by admixing a crude melt of ethylene urea with water.

5. The process according to at least one of claims 1 to 4, wherein the breaking-up apparatus is a flaking roller or a flaking belt.

6. The process according to at least one of claims 1 to 4, wherein the breaking-up apparatus is a palletizing belt.

## Revendications

1. Procédé pour la production d'éthylène-urée sous forme solide, dans lequel on refroidit au moyen d'un dispositif d'isolement une masse fondue de produit éthylène-urée ayant une teneur en eau de 5 à 15 % en poids et la matière solide obtenue présente essentiellement la même teneur en eau que la masse fondue de produit.

2. Procédé selon la revendication 1, **caractérisé en ce que** la masse fondue de produit et la matière solide présentent une teneur en eau de 8 à 12 % en poids.

3. Procédé selon la revendication 1 ou 2, **caractérisé en ce qu'**on obtient la masse fondue de produit contenant de l'eau en faisant fondre de l'éthylène-urée cristallisée.

4. Procédé selon la revendication 1 ou 2, **caractérisé en ce qu'**on obtient la masse fondue de produit contenant de l'eau en ajoutant de l'eau à une masse fondue brute de l'éthylène-urée.

5. Procédé selon au moins l'une quelconque des revendications 1 à 4, **caractérisé en ce que** pour ce qui concerne le dispositif d'isolement il s'agit d'un cylindre à lamelles ou d'une bande à lamelles.

6. Procédé selon au moins l'une quelconque des revendications 1 à 4, **caractérisé en ce que** pour ce qui concerne le dispositif d'isolement il s'agit d'une bande de pastillage.
